# EUROPÄISCHE PATENTSCHRIFT

(11) **EP 2 023 832 B1**
(45) Veröffentlichungstag und Bekanntmachung des Hinweises auf die Patenterteilung: **14.07.2010**
(21) Anmeldenummer: 07724808.6
(22) Anmeldetag: 03.05.2007
(51) Int. Cl.: A61B 17/34, A61B 17/32

(54) **CHIRURGISCHER OBTURATOR**
SURGICAL OBTURATOR
OBTURATEUR CHIRURGICAL

(30) Priorität: 27.05.2006 DE 102006024756
(43) Veröffentlichungstag der Anmeldung: 18.02.2009
(73) Patentinhaber: Aesculap AG, 78532 Tuttlingen (DE)
(72) Erfinder: MALIGLOWKA, Johann, 78600 Kolbingen (DE); MAYENBERGER, Rupert, 78239 Rielasingen (DE); SCHWEITZER, Tom, 78532 Tuttlingen (DE)
(74) Vertreter: Hoeger, Stellrecht & Partner Patentanwälte
(86) Internationale Anmeldenummer: PCT/EP2007/003882
(87) Internationale Veröffentlichungsnummer: WO 2007/137673

(56) Entgegenhaltungen:
- EP-A- 0 495 633
- DE-U1-202006 008 406
- DE-U1-202006 018 883
- US-A- 5 591 191
- US-A- 5 609 604
- US-A- 5 662 673
- US-A1- 2003 100 914

## Beschreibung

Die Erfindung betrifft einen chirurgischen Obturator zum Durchstechen einer Körperwand mit einem Messer, dessen Schneide von einer Spitze des Obturators nach verschiedenen Seiten nach außen und gegenüber der Spitze zurückspringend verläuft und das über seine gesamte Länge parallel zur Durchstechrichtung orientiert ist, so dass die Schneide in Durchstechrichtung weist.

Obturatoren dieser Art werden verwendet, um eine Öffnung in einer Körperwand auszubilden, durch die eine Kanüle oder ein Rohr in das Körperinnere eingeführt werden kann, beispielsweise zur Vorbereitung einer minimalinvasiven Operation.

Es sind Obturatoren bekannt mit in Durchstechrichtung weisenden, von der Spitze nach außen und zurückspringend verlaufenden Schneiden, bei denen die Messerschneide eine V-Form aufweist, also mit geradlinigen Schneiden, die gegenüber einer durch die Spitze des Obturators laufenden Längsachse desselben geneigt sind. Mit derartigen Obturatoren können saubere Schnitte durchgeführt werden, wenn der Obturator durch die Körperwand vorgeschoben wird, es ist aber schwierig, diesen Durchstechprozess so gefühlvoll durchzuführen, dass nach dem Durchstechen innere Organe nicht unbeabsichtigt verletzt werden. Wenn der Schneidvorgang einmal eingeleitet ist, schreitet dieser mit relativ geringem Widerstand fort, so dass der Chirurg nicht genau feststellen kann, wie weit der Obturator bereits in die Körperwand eingedrungen ist.

Das Dokument US 5662673 A offenbart einen Obturator gemäß dem einleitenden Teil von Anspruch 1.

Es ist Aufgabe der Erfindung, einen gattungsgemäßen chirurgischen Obturator so auszubilden, dass mit ihm gefühlvolleres Durchstechen einer Körperwand möglich ist.

Diese Aufgabe wird bei einem chirurgischen Obturator der eingangs beschriebenen Art erfindungsgemäß dadurch gelöst, dass die Schneide schraubenlinienförmig verläuft. Bei einem solchen Verlauf erfolgt der Durchstechvorgang nicht allein durch ein axiales Vorschieben des Obturators, sondern durch eine Überlagerung einer Vorschubbewegung und einer Drehbewegung ähnlich der Bewegung beim Einschrauben einer selbstschneidenden Knochenschraube. Eine solche überlagerte Vorschub- und Drehbewegung ist wesentlich kontrollierter ausführbar als eine reine Vorschubbewegung, der Operateur kann auch an dem Drehwinkel abschätzen, wie weit das Messer bereits in die Körperwand eingedrungen ist.

Es ist vorteilhaft, wenn die Abschnitte des Messers auf verschiedenen Seiten der Spitze durch eine Drehung um eine durch die Spitze des Obturators hindurchgehende Längsachse ineinander überführbar sind, wenn also ähnliche Geometrien der Abschnitte des Messers verwendet werden, die sich lediglich durch ihre Winkelstellung unterscheiden.

Insbesondere kann vorgesehen sein, dass die Projektion der Schneide auf eine senkrecht zur Längsachse des Obturators stehende Ebene ausgehend von der Spitze einen Kreisbogen oder zumindest annähernd einen Kreisbogen bildet.

Dabei ist es vorteilhaft, wenn sich der Kreisbogen von der Spitze aus über einen Winkel von etwa 180° erstreckt, also ungefähr über einen Halbkreis.

Die Abschnitte der Schneiden gehen auf verschiedenen Seiten der Spitze ohne Knick ineinander über, so daß die Schneide im Bereich der Spitze des Obturators quer zur Längsachse des Obturators verläuft. Günstig ist es, wenn der Obturator eine sich verjüngende Einführfläche an seinem einen Ende trägt mit einem Schlitz, durch den das Messer heraussteht. Das Messer ist also durch die Einführfläche weitgehend abgedeckt, lediglich die Schneiden stehen aus dem Schlitz geringfügig hervor, und die Einführfläche legt sich an das Gewebe der Körperwand an, wenn der Obturator unter Ausbildung einer Durchstechöffnung eingedreht und vorgeschoben wird. Dies führt zu einer guten Führung und schließt gleichzeitig die entstehende Öffnung in der Körperwand ab, gegebenenfalls sogar gasdicht.

Die Einführfläche kann beispielsweise annähernd kegelstumpfförmig ausgebildet sein und eine abgerundete Spitze aufweisen.

Die nachfolgende Beschreibung bevorzugter Ausführungsformen der Erfindung dient im Zusammenhang mit der Zeichnung der näheren Erläuterung. Es zeigen:

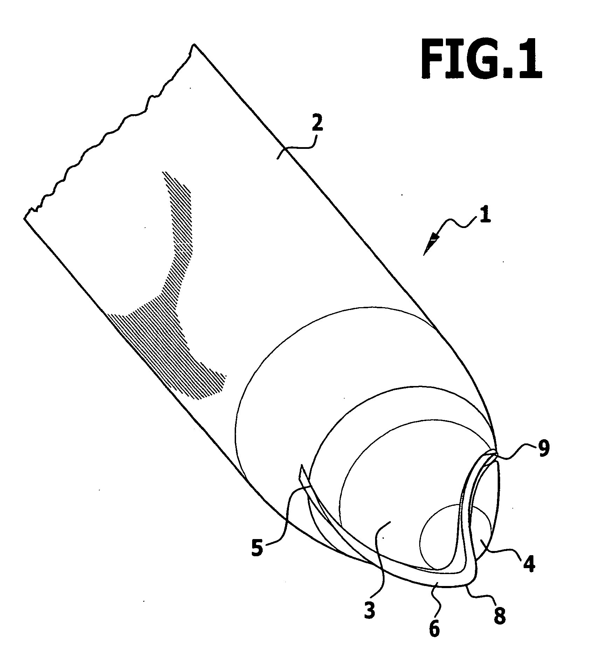

- Figur 1:: eine perspektivische Ansicht des Einstechbereichs eines Obturators mit schraubenförmiger Schneidengeometrie;

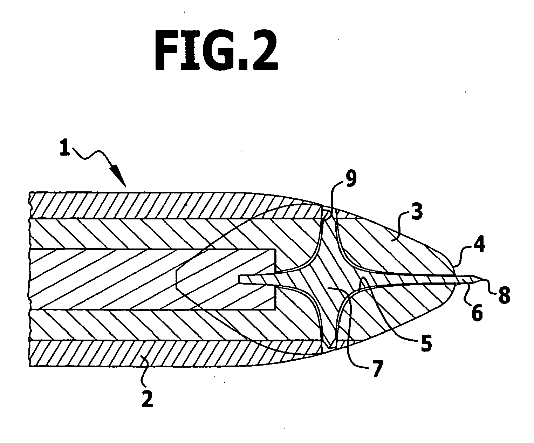

- Figur 2:: eine Längsschnittansicht durch den Einstechbereich des Obturators der Figur 1 längs Linie 2-2 in Figur 3;
- Figur 3:: eine Ansicht des Obturators der Figur 1 von der Spitze aus gesehen;
- Figur 4:: eine schematische Darstellung eines Messers für den Obtu- rator der Figuren 1 bis 3 und
- Figuren 5 bis 8:: unterschiedliche Anordnungen der Schneiden im Bereich der Spitze des Obturators, wobei nur die in Figur 6 dargestellte Anordnung unter die vorliegenden Ansprüche fällt.

Der in der Zeichnung dargestellte Obturator 1 umfasst ein rohrschaftförmiges Gehäuse 2 mit einer annähernd kegelstumpfförmigen Einführfläche 3 an einem Ende, die von der Außenwand des schaftförmigen Gehäuses 2 kontinuierlich zu einer Spitze führt. Diese Spitze 4 ist abgerundet.

In der Einführfläche 3 ist ein Schlitz 5 angeordnet, der durch die Spitze 4 hindurchgeht und der sich parallel zur Längsachse des Obturators 1 erstreckt.

Durch diesen Schlitz 5 ragt ein Messer 6 hindurch, das Teil eines Messerkörpers 7 ist, der im Inneren des Gehäuses 2 und unter der Einführfläche 3 angeordnet ist. Das Messer 6 ragt geringfügig aus dem Schlitz 5 heraus, wobei die Schneide 8 des Messers 6 im Wesentlichen parallel zu dem Schlitz 5 verläuft.

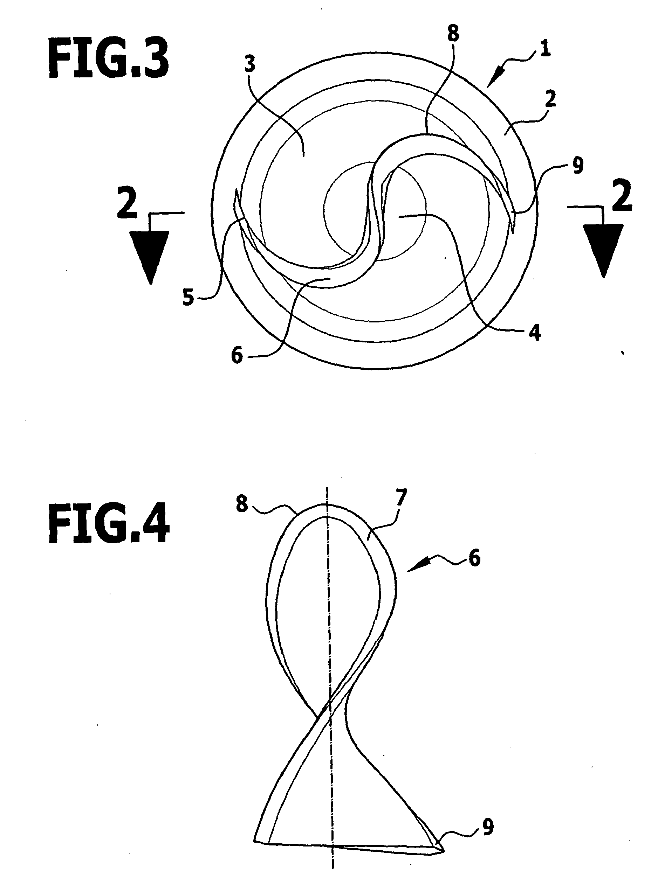

Die Schneide 8 verläuft dabei schraubenförmig, das heißt sie verläuft von der Spitze 4 ausgehend auf einer sich erweiternden Schraubenlinie nach außen und zum schaftförmigen Gehäuse 2 hin. Die Projektion der schraubenlinienförmigen Schneide 8 auf eine senkrecht zur Längsachse des Obturators verlaufende Ebene ist somit S-förmig, wie dies in Figur 3 dargestellt ist. Ausgehend von der Spitze 4 der Schneide ist diese Projektion etwa ein Halbkreis, wobei das von der Spitze 4 entfernte Ende 9 noch im Bereich der Einführfläche 3 endet, also in einem radialen Abstand von der Spitze 4, der kleiner ist als der Radius des schaftförmigen Gehäuses 2. Die Schneide 8 erstreckt sich dabei über etwa 180°, und die beiden Abschnitte der Schneide 8 auf gegenüberliegenden Seiten der Spitze 4 gehen im Bereich der Spitze 4 ohne Knick ineinander über, das heißt die Schneide verläuft im Bereich der Spitze 4 quer zur Längsachse des Obturators.

Die beiden auf gegenüberliegenden Seiten der Spitze 4 liegenden Abschnitte der Schneide sind dabei gleich ausgebildet, so dass diese Abschnitte durch eine Drehung um 180° um die Längsachse des Gehäuses 2 zur Deckung gebracht werden können.

Wenn dieser Obturator 1 in Richtung seiner Längsachse an eine Körperwand herangeschoben wird, in der eine Öffnung erzeugt werden soll, legt sich die Schneide 8 zunächst im Bereich der Spitze 4 an die Außenseite der Körperwand und bringt dort einen horizontalen Schnitt ein. Beim weiteren Vorschieben dreht der Operateur gleichzeitig den Obturator 1 um seine Längsachse, so dass die schrauben- oder helixförmige Schneide 8 ähnlich wie eine selbstschneidende Knochenschraube mit in Einschraubrichtung zunehmendem Durchmesser der Schraubgänge in die Körperwand eindringt und dabei den erzeugten Schnitt kontinuierlich erweitert, bis der Schnitt eine Länge erreicht hat, die durch die Gesamtlänge der Schneide 8 vorgegeben ist. Damit ist der Schneidvorgang beendet, die Einführfläche 3 ist jedoch noch nicht vollständig durch die erzeugte Öffnung hindurchgetreten, da ihr Außendurchmesser am gehäuseseitigen Ende größer ist. Die Einführfläche 3 verschließt daher die Öffnung dicht, durch weiteres Eindrücken der Einführfläche 3 kann diese Öffnung aufgeweitet werden, wobei das Gewebe der Körperwand dann dichtend an der Einführfläche 3 entlang gleitet und am Gehäuse 2 anliegt.

Durch den schraubenlinienförmigen Verlauf der Schneide 8 muss der Obturator 1 zum Erzeugen der Durchstechöffnung neben der Vorschubbewegung auch eine Drehbewegung erfahren, und diese überlagerte Bewegung aus Vorschub und Drehung ermöglicht dem Operateur ein wesentlich gefühlvolleres Durchstechen der Körperwand als bei einer Schneide, die geradlinig verläuft und die daher nur einen Vorschub des Obturators 1 zulässt.

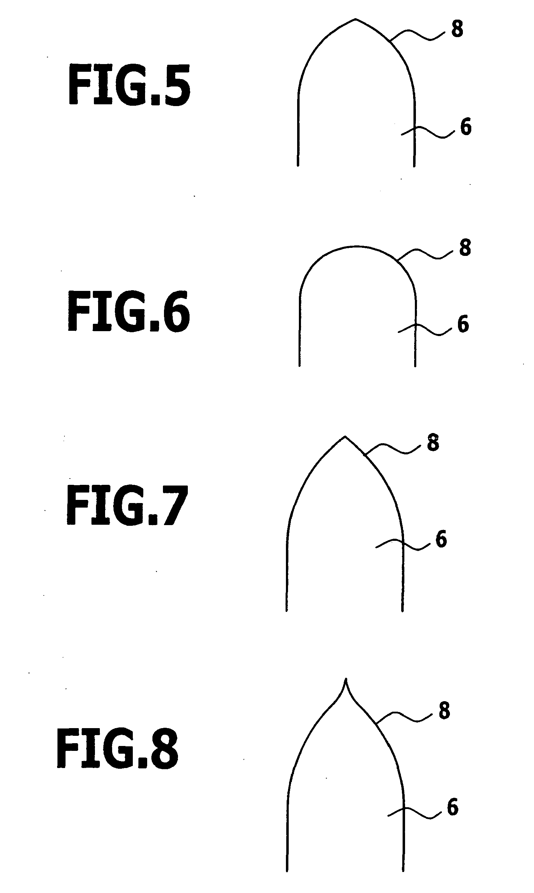

Die Abschnitte der Schneide 8 auf gegenüberliegenden Seiten der Spitze 4 gehen ohne Knick ineinander über, es ergibt sich dann von der Seite aus gesehen eine Kontur gemäß Figur 6.

Bei bem Obturator gemäß Fig.5 oder 7 treffen sich die Abschnitte der Schneide 8 in der Spitze 4 in einem spitzen Winkel.

Bei einem Obturator gemäß Figur 8 ist vorgesehen, dass die beiden Abschnitte der Schneide 8 im Bereich der Spitze 4 eine Zentrierspitze ausbilden, die dem Operateur das Ansetzen des Obturators an die Körperwand erleichtert.

Die Obturatoren der Figuren 5, 7 und 8 fallen nicht unter die vorliegenden Ansprüche.

## Patentansprüche

1. Chirurgischer Obturator (1) zum Durchstechen einer Körperwand mit einem Messer (6), dessen Schneide (8) von einer Spitze des Obturators nach verschiedenen Seiten schraubenlinienförmig nach außen und gegenüber der Spitze zurückspringend verläuft und das über seine gesamte Länge parallel zur Durchstechrichtung orientiert ist, so dass die Schneide in Durchstechrichtung weist, wobei die Projektion der schraubenlinienförmigen Schneide (8) auf eine senkrecht zur Längsachse des Obturators verlaufende Ebene S-förmig ist, wobei die Abschnitte des Messers (6) auf verschiedenen Seiten der Spitze (4) durch eine Drehung um 180° um die Längsachse des Obturators ineinander überführbar sind,
**dadurch gekennzeichnet, dass** die beiden Abschnitte der Schneide (8) auf gegenüberliegenden Seiten der Spitze (4) im Bereich der Spitze (4) ohne Knick ineinander übergehen, so dass die Schneide im Bereich der Spitze (4) quer zur Längsachse des Obturators verläuft.

2. Obturator nach Anspruch 1, **dadurch gekennzeichnet, dass** die Projektion der Abschnitte der Schneide (8) auf eine senkrecht zur Längsachse des Obturators (1) stehende Ebene ausgehend von der Spitze (4) einen Kreisbogen bildet.

3. Obturator nach Anspruch 2, **dadurch gekennzeichnet, dass** sich der Kreisbogen von der Spitze (4) aus über einen Winkel von etwa 180° erstreckt.

4. Obturator nach einem der voranstehenden Ansprüche, **dadurch gekennzeichnet, dass** er eine sich verjüngende Einführfläche (3) an seinem einen Ende trägt mit einem Schlitz (5), durch den das Messer (6) hervorsteht.

5. Obturator nach Anspruch 4, **dadurch gekennzeichnet, dass** die Einführfläche (3) annähernd kegelstumpfförmig ausgebildet ist und eine abgerundete Spitze (4) aufweist.

## Claims

1. Surgical obturator (1) for puncturing a body wall with a knife (6), the cutting edge (8) of which extends in the shape of a helical line outwards from a tip of the obturator in the direction of various sides and backwards in relation to the tip, and which is orientated throughout its entire length parallel to the puncturing direction, so that the cutting edge points in the puncturing direction, the projection of the helical line-shaped cutting edge (8) onto a plane extending perpendicularly to the longitudinal axis of the obturator being S-shaped, and the sections of the knife (6) at various sides of the tip (4) being transferable into each other's position by rotation through 180° about the longitudinal axis of the obturator, **characterized in that** the two sections of the cutting edge (8) at opposite sides of the tip (4) merge without any sharp bend in the region of the tip (4), so that in the region of the tip (4) the cutting edge extends transversely to the longitudinal axis of the obturator.

2. Obturator in accordance with claim 1, **characterized in that** the projection of the sections of the cutting edge (8) onto a plane extending perpendicularly to the longitudinal axis of the obturator (1), starting from the tip (4), forms the arc of a circle.

3. Obturator in accordance with claim 2, **characterized in that** the arc of the circle extends from the tip (4) over an angle of approximately 180°.

4. Obturator in accordance with any one of the preceding claims, **characterized in that** it bears at one end thereof a tapering insertion area (3) with a slot (5) through which the knife (6) projects.

5. Obturator in accordance with claim 4, **characterized in that** the insertion area (3) is of approximately frustoconical configuration and has a rounded tip (4).

## Revendications

1. Obturateur chirurgical (1) pour transpercer ou perforer une paroi corporelle à l'aide d'une lame coupante (6) dont le tranchant de coupe (8) s'étend, à partir d'une pointe de l'obturateur, selon différents côtés vers l'extérieur, sous forme de ligne hélicoïdale et en retrait par rapport à la pointe, la lame coupante étant orientée, sur la totalité de sa longueur, parallèlement au sens de perforation, de sorte que le tranchant de coupe est dirigé dans le sens de la perforation, la projection du tranchant de coupe (8) en forme de ligne hélicoïdale sur un plan qui s'étend perpendiculairement à l'axe longitudinal de l'obturateur, présentant une forme en S, et les tronçons de la lame coupante (6) sur des côtés différents de la pointe (4), pouvant être transposés l'un dans l'autre par une rotation de 180° autour de l'axe longitudinal de l'obturateur,
**caractérisé en ce que** les deux tronçons du tranchant de coupe (8) sur des côtés opposés de la pointe (4), se raccordent l'un à l'autre dans la région de la pointe. (4), sans brisure ou discontinuité, de sorte que le tranchant de coupe, dans la région de la pointe (4), s'étend transversalement à l'axe longitudinal de l'obturateur.

2. Obturateur selon la revendication 1, **caractérisé en ce que** la projection des tronçons du tranchant de coupe (8) sur un plan perpendiculaire à l'axe longitudinal de l'obturateur (1), forme un arc de cercle à partir de la pointe (4).

3. Obturateur selon la revendication 2, **caractérisé en ce que** l'arc de cercle s'étend à partir de la pointe (4) sur un angle d'environ 180°.

4. Obturateur selon l'une des revendications précédentes, **caractérisé en ce qu'**à l'une de ses extrémités, il porte une surface d'introduction (3), qui se rétrécit et comporte une fente (5) de laquelle dépasse vers l'extérieur la lame coupante (6).

5. Obturateur selon la revendication 4, **caractérisé en ce que** la surface d'introduction (3) est d'une configuration sensiblement de forme tronconique, et présente une pointe (4) arrondie.
